# EUROPEAN PATENT SPECIFICATION

(11) **EP 4 125 368 B1**
(45) Date of publication and mention of the grant of the patent: **10.04.2024**
(21) Application number: 21717357.4
(22) Date of filing: 31.03.2021
(51) Int. Cl.: A01N 25/04, A01N 25/14, A01N 59/16, A01N 59/20, A61P 31/02

(54) **A MODIFIED BENTONITE, A COMPOSITION BASED ON A MODIFIED BENTONITE AND A METHOD FOR MANUFACTURING THEREOF**
MODIFIZIERTER BENTONIT, ZUSAMMENSETZUNG AUF BASIS EINES MODIFIZIERTEN BENTONITS UND VERFAHREN ZUR HERSTELLUNG DAVON
BENTONITE MODIFIÉE, COMPOSITION À BASE DE BENTONITE MODIFIÉE ET SON PROCÉDÉ DE FABRICATION

(30) Priority: 01.04.2020 PL 43342320
(43) Date of publication of application: 08.02.2023
(73) Proprietor: PRZYBYSZ, Kazimierz, 93-322 Lodz (PL)
(72) Inventor: PACZKOWSKA, Joanna, 32-400 Myslenice (PL); PRZYBYSZ, Kazimierz, 93-322 Lodz (PL)
(74) Representative: Kancelaria Eupatent.pl Sp. z.o.o
(86) International application number: PCT/EP2021/058467
(87) International publication number: WO 2021/198348

(56) References cited:
- WO-A1-2010/130823
- WO-A1-2011/073193
- WO-A1-2011/131422
- WO-A2-2011/070175

## Description

### TECHNICAL FIELD

The present disclosure relates to a modified bentonite having biostatic and biocidal properties, a composition containing a modified bentonite, and a method of manufacturing thereof.

### BACKGROUND

Bentonite is a mineral of volcanic origin, classified as a clay material. The main ingredient of bentonite is montmorillonite, however, it is assumed that bentonite is a clay rock containing not less than 75% montmorillonite, and minerals with a content of 50 to 75% montmorillonite are commonly referred to as bentonite clays. Due to its high content, montmorillonite affects the main properties of bentonite. It is assumed that the bentonite contain three-layer packets having a small electric charge compensated by inter-packet cations that are interchangeable. The distance of adjacent packets in the bentonite structure depends on the type of inter-packet cations contained therein, and generally the greater the radius of the cation, the greater the distance of adjacent packets. The cation sorption capacity for bentonite is relatively high. Naturally occurring bentonites typically have, in their interchangeable positions, calcium cations: Ca²⁺ and/or magnesium cations: Mg²⁺, whereby due to weak chemical interactions, it is possible to exchange them with other ions, such as sodium (Na⁺) and/or potassium (K⁺) ions. Bentonite that contains sodium and/or potassium ions as inter-packet cations is called activated bentonite, abbreviated as Na-bentonite, K-bentonite, Na/K-bentonite, respectively, and the process of obtaining such bentonite is called activation. Typically, the activation of bentonite is carried out by mixing bentonite with appropriate sodium or potassium carbonates.

Bentonite is a relatively cheap raw material and is harmless to the natural environment. Due to the ease of ion exchange in activated bentonite, other ions can be introduced in place of sodium ions, and this process is called intercalation and leads to the obtaining of modified, i.e. intercalated with a selected ion, bentonite. For example, powders of bentonite intercalated with cations of a selected type of metal are known, which ensures that the bentonite obtains appropriate properties, for example, the powder of bentonite intercalated with silver ions shows a bactericidal effect, and the powder of bentonite intercalated with copper ions shows a fungicidal effect, therefore these powders are mixed to obtain a powder mixture showing bactericidal and fungicidal activity with biocidal effectiveness being the sum of the effectiveness of individual powders.

In the patent literature there are various methods of modifying bentonite in order to obtain its biocidal properties.

WO2011131422 discloses a toilet soap with prolonged biocidal properties. The soap contains an additive of chemically modified bentonite in a powdered form, which constitutes a soap ingredient having biocidal properties. The described method of modifying bentonite consists in activating bentonite with sodium ions (Na⁺) to obtain an activated bentonite: Na-bentonite, i.e. in the form of montmorillonite. One portion of Na-bentonite is mixed with an aqueous solution of silver nitrate to obtain bentonite in the form of a powder modified with silver ions (Ag⁺). In turn, the second portion of activated bentonite is mixed with an aqueous solution of copper (II) sulphate (VI) to obtain bentonite in the form of a powder modified with copper ions (Cu²⁺). Then, both powders of bentonite intercalated with silver ions and bentonite intercalated with copper ions are mixed in appropriate proportions so as to obtain a powder mixture with an appropriate silver to copper ratio of e.g. 1:1 or 1:5. According to this document, such proportions ensure an appropriate biocidal activity of the powder mixture while maintaining the economic viability of the product. Thus, the individual bentonite grains in each powder are intercalated with only one type of ion: Ag⁺ or Cu²⁺.

WO2010/130823 discloses a biocide with antifungal and antibacterial properties for use in construction and medicine. Biocide, as an active ingredient, contains a mixture of bentonite powders with nanometer grain size. The method of modifying bentonite consists in manufacturing each type of powder separately. The following are separately manufactured: bentonite powder with zinc ions (Zn²⁺), bentonite powder with silver ions (Ag⁺), and optionally also bentonite powder with copper ions (Cu²⁺). The powders are then mixed in appropriate proportions in order to obtain a mixture of two or three different bentonite powders. Therefore, the resulting mixture of powders contains such bentonite grains that the individual bentonite grain being an ingredient of this mixture contains only zinc ions, or only copper ions, or only silver ions.

WO2011/070175 discloses a method in which three different bentonite powders are manufactured separately, each powder having a grain size in the range of 20 to 150 nm, including a powder with bentonite grains containing only zinc ions, a powder with bentonite grains containing only copper ions and a powder with bentonite grains containing only silver ions. These powders are then mixed together to form a mixture with fungicidal and bactericidal properties. The method of manufacturing each of the powders involves mixing the activated bentonite with an aqueous salt solution containing ions of only one metal that is to be intercalated in bentonite grains, the mixing being carried out using ultrasonic waves with a frequency in the range of 20 - 50 kHz and an intensity in the range of 10 - 100 WT/cm², which reduces the preparation time of each powder.

WO2011/035988A1 discloses an antiseptic ointment composition containing a mixture of bentonite powders with grain sizes in the nanometer range. Each powder that is an ingredient in this mixture contains bentonite grains intercalated with only one type of metal ions: Ag⁺ or Cu²⁺, or Zn²⁺. Such a mixture of several different bentonite powders gives the ointment antibacterial and antifungal properties.

WO2011107311 discloses a hygiene product, such as: paper towels, napkins, diapers or toilet paper, containing dried cellulose with the addition of a mixture of two different bentonite powders, each with particle size below 100 nm. Each powder that is an ingredient in this mixture contains bentonite grains intercalated with only one type of metal: in the form of silver ions or copper ions. On the other hand, hygienic products are made of cellulose pulp with the addition of these two bentonite powders, with a cellulose pulp density of 100 - 800 kg/m³, with the addition of a copolymer based on a modified protein - as a binding agent.

RU2371156 discloses a cellulose-based sanitary product with biocidal properties, including antibacterial and antifungal properties. These products may be in the form of diapers or toilet towels containing at least one cellulose layer with the mentioned biocidal properties. The method of manufacturing these products consists, first of all, in manufacturing a hydrosol containing a mixture of different bentonite powders: including bentonite powder modified with silver ions - i.e. with grains intercalated with silver ions, and bentonite powder modified with copper ions - i.e. with grains intercalated with copper ions. The mixture of these two powders contains a total of 2 to 8 wt.% of metals. However, the hydrosol with the mixture of bentonite powders is introduced to the cellulose pulp in the amount necessary to obtain a concentration of the mixture of bentonite powders in the cellulose pulp of not more than 2%.

WO2011073193 describes a method for antiseptic processing of the surface of a product made of organosilicon rubbers with a molecular weight of 2·10⁵-6·10⁵. this method consists in the formation of a coating of antiseptic properties i.a. due to the use of bentonite powder having particles of 150 nm or less serving as a biocide.

### SUMMARY

In the prior are patent documents as discussed in the background, a modified bentonite is defined as a bentonite containing one or more intercalated ions, including silver (Ag⁺), copper (Cu²⁺), zinc (Zn²⁺) ions. It should be noted, however, that the solutions used in these documents relate in each case to mixtures of several different bentonite powders. Thus, each of the described mixtures actually contains such bentonite grains, wherein each single (individual) grain in intercalated with ions of only a single type of metal: either silver ions (Ag⁺) or copper ions (Cu²⁺) or zinc ions (Zn²⁺). This follows from that each of these documents describes only such methods of obtaining the modified bentonite, wherein individual powders are manufactured separately by mixing bentonite with either silver nitrate or copper sulphate or zinc sulphate. Then, the obtained powders are combined together by mixing them in specific proportions, as clearly concluded after analysing the embodiments described in these documents. None of the cited documents mentions a synergistic effect that could result from a combination of individual metal ions in a single grain of the modified bentonite.

The prior art patent documents also indicate that the methods of modifying bentonite are subject to continuous modifications, including mixing two or three different bentonite powders - each powder consisting of bentonite grains, wherein each single bentonite grain is intercalated with only a single type of metal ion, such as Ag⁺ or Cu²⁺, or Zn²⁺. These powders are mixed in specific proportions in order to obtain a bentonite mixture with a specific proportion of bentonite grains containing a particular type of metal ion. When manufacturing this type of bentonite mixtures, through selection of appropriate metal ions, it is aimed to improve the demonstrated biocidal properties of the mixture and, simultaneously, to increase the process efficiency of manufacturing individual powders, as well as to reduce raw material costs. Mixtures of several different bentonite powders are used in more and more new products, including cellulose-based hygiene products, cosmetics, everyday utensils, furniture boards or construction articles, in order to give them biocidal properties.

Therefore, there is a need for further development of bentonite products in order to improve its biostatic and biocidal properties.

There is disclosed a modified bentonite, a composition comprising the modified bentonite, and a method for manufacturing the modified bentonite according to the appended claims.

The method may be used for obtaining a paste of the modified bentonite, the method of paste preparation comprising: mixing each of a plurality of dry bentonite grains of a diameter not exceeding 33 microns with water in amount 0,5 to 1 liter of water per each 100 g of dry bentonite grains, wherein the mixing is conducted with a stirrer at steering speed from 0.4 to 2.0 revolution per second, at a temperature not exceeding 50°C, to obtain a slurry; feeding the obtained slurry with water solutions of two different metal nitrates whilst mixing the slurry with the stirrer at steering speed from 0.4 to 2.0 revolution per second for 30 to 60 minutes at a temperature not exceeding 50°C to obtain a paste of the modified bentonite.

At the feeding, the slurry can be fed with the water solution comprising silver (I) nitrate (V) and the water solution comprising: copper (II) nitrate (V), zinc (II) nitrate (V), tin (II) nitrate (V) or nickel (II) nitrate (V).

The water solution of silver (I) nitrate (V) may further comprise silver (I) chloride (V) and nitric acid (HNOs).

The obtained paste of the modified bentonite may comprise from 8 to 12% of the modified bentonite (on a dry weight basis).

The obtained paste of the modified bentonite may be further diluted with water to obtain a slurry of concentration 3% of the modified bentonite (on a dry weight basis). The slurry may be applied on a substrate using a spray coating technique.

### BRIEF DESCRIPTION OF DRAWINGS

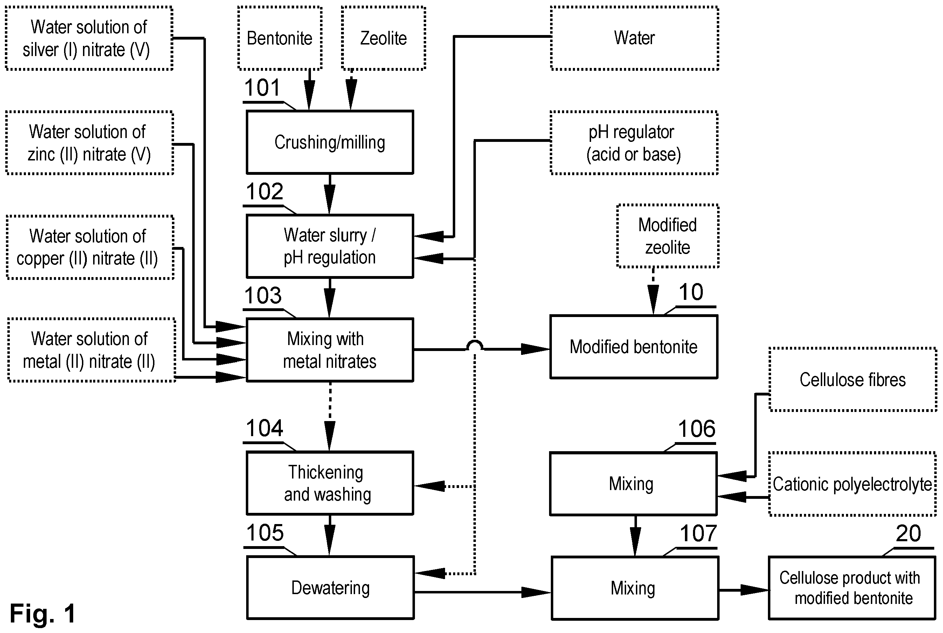

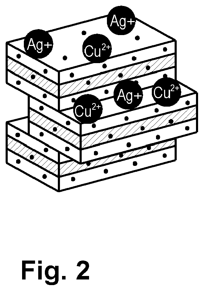

The present disclosure is shown in an embodiment in a drawing, in which: Fig. 1 schematically shows a method of manufacturing a modified bentonite with improved biocidal and biostatic properties; Fig. 2 shows a pictorial representation of a portion of bentonite grain intercalated with two different metal ions: silver and copper ions, in its interchangeable positions.

### DETAILED DESCRIPTION

Metal cations having a valence of two will be referred herein to as dipositive metal cations or divalent metal cations or divalent/dipositive metal ions.

A method for manufacturing modified bentonite includes mixing bentonite, preferably activated bentonite containing intercalated sodium (Na⁺) and/or potassium (K⁺) ions with nitrogen salts of at least two different metals, the metals in their salts being in the form of respective cations selected from the group consisting of: silver (I) ion Ag (⁺) and dipositive metal ions (Me²⁺), preferably selected from the group consisting of: copper (II) ions (Cu²⁺), zinc (II) ions (Zn²⁺), tin (II) ions (Sn²⁺), zircon (II) ions (Zr²⁺), nickel (II) ions (Ni²⁺), bismuth (II) ions (Bi²⁺) and cobalt (II) ions (Co²⁺).

As a result of the conducted method, a modified bentonite is obtained having a form of intercalated bentonite grains, wherein each single intercalated bentonite grain is intercalated with at least two different metal ions selected from the group consisting of silver ions (Ag⁺) and dipositive metal ions (Me²⁺). In consequence, the obtained modified bentonite containing intercalated bentonite grains that have at least two different metal ions in a single grain, shows improved biocidal properties, including even a several-fold increase in biocidal activity, depending on the type of metal ions contained in a single grain of the modified bentonite.

Bentonite, and more preferably activated bentonite, including bentonite intercalated with sodium (Na⁺) and/or potassium (K⁺) ions, is used as a raw material in the process. The type of ions contained in the structure of activated bentonite determines the size of the inter-packet gap in the bentonite structure. Activated bentonite: Na/K-bentonite is characterised by larger inter-packet gaps in its structure, thanks to which, in the case of its use, inter-packet ion exchange, i.e. bentonite modification, occurs faster than in the case of bentonite activated only with sodium (Na⁺) ions (Na-bentonite) with a smaller ion radius than potassium ions: K⁺. For example, commercially available Na/K-bentonite can be used in the developed method.

Furthermore, natural, unactivated bentonite can be used in the method presented herein as a natural raw material, containing inter-packet Ca²⁺ and/or Mg²⁺ ions, but in this case a product with less efficient biocidal properties is obtained.

Furthermore, as shown in Fig. 1, optionally a mixture of bentonite and zeolite, or activated bentonite and zeolite, preferably in weight proportions of bentonite or activated bentonite : zeolite in the range of 50:1 to 5:1, may be used as a starting raw material in the developed method.

In order to produce the modified bentonite with biocidal properties, as schematically shown in Fig. 1, the starting raw material: bentonite or more preferably bentonite modified with sodium and/or potassium ions, and most preferably: Na/K-bentonite, or optionally its mixture with zeolite, is comminuted in step 101 until the desired particle size is obtained, in case it has not been comminuted before. The comminution can be carried out by means of various devices, for example ball mills. Preferably, bentonite is comminuted to a powder with a particle size in the range of 200 nm to 50 µm. The size of the bentonite comminution affects the biostatic and biocidal properties of the finished product. Along with the reduction of the bentonite grain diameter, the contact surface of this grain with potential pathogens - in the finished product - increases, and the probability of interaction of two different metal ions contained in the bentonite grain with the pathogen is higher. For bentonite grains with a minimum size of 200 nm, a synergistic effect of the presence of two different metal ions intercalated in a single grain is also observed, which is manifested by a significant increase in its biocidal properties. Grains of this size do not penetrate the mucous membranes into the human body, so it can be safely used as an additive to various hygiene products, including cosmetics, without any risk of negative effects on the human body. Furthermore, grains of modified bentonite with a maximum size of up to 50 µm are characterised by better adhesion to various surfaces, thanks to which products sprayed with modified bentonite show more permanent biostatic and biocidal properties. The grain size of the optionally used zeolite is in the same range as that of the bentonite.

In step 102, form the comminuted bentonite, an aqueous suspension with a pH preferably in the range of 6 to 9 depending on the type of bentonite is manufactured. The pH value is adjusted by adding an acid or a base, for example: HNO₃, CH₃COOH or KOH. Then, in step 103, aqueous solutions of salts of at least two different metals selected from the group of: Ag⁺, and Me²⁺ are introduced to the manufactured suspension. In step, as dipositive metal cations Me²⁺, the following are preferably used: Zn²⁺, Cu²⁺, Sn²⁺, Zr²⁺, Ni²⁺, Bi²⁺ or Co²⁺. Preferably, metal ions are introduced into the aqueous suspension in the form of nitrates (V), the advantage of which is that the product can be easily purified from nitrates at a later stage in the process. Nitrates, which constitute contamination of the finished product, can be removed by washing with water or by heating the reaction product at a temperature preferably in the range of 150-200°C, in which nitrates are decomposed and are released from the product in the form of oxides. In additional, all salts of nitric acid (V) are highly soluble so that one cannot observe any precipitation in the reaction mixture, which would change the progress of ion exchange - intercalation and the composition of the final product.

In step 103, salts of individual metals in the form of aqueous solutions, including for example: silver (I) nitrate (V), zinc (II) nitrate (V), copper (II) nitrate (V), tin (II) nitrate (V) ), zirconium (II) nitrate (V), nickel (II) nitrate (V), cobalt (II) nitrate (V) can be introduced into the aqueous suspension of bentonite. In the case of balanced amounts of cations for intercalation of bentonite grains, their salt solutions may be administered together or in any order under constant mixing. However, more preferably, the aqueous salt solutions are introduced in a selected order: a solution of salt of one metal is introduced after completing the introduction of a solution of salt of the other metal, which ensures a more uniform concentration of metal cations in the solution. In the case of using much smaller amounts of silver cations intecalted in bentonite, it is best to start the ion exchange by adding a solution of silver (I) nitrate (I) at an appropriate concentration, and then solutions of the other cations until full or partial saturation according to the determined degree of exchange is obtained. Continuous mixing in step 103 ensures a good distribution of all metal ions throughout the volume of the mixture, thereby each grain of bentonite is intercalated with all types of metal ions that are present in this mixture. In other words: a single grain of bentonite is hetero-intercalated as a result of the conducted method. In Fig. 2 in the form of a pictorial representation, as an embodiment, it is shown a portion of bentonite grain intercalated with two different metal ions: silver and copper ions, in its interchangeable positions.

In order to obtain optimal saturation of the bentonite grains with metal ions, the following proportions of bentonite and at least two different metal nitrates (V) can preferably be used in the following amounts: 0.001 mole of silver (I) nitrate (V) per 1 kg of bentonite and/or 0.005 mole of zinc (II) nitrate (V) per 1 kg of bentonite, and/or 0.001 mole of copper (II) nitrate (V) per 1 kg of bentonite, and/or 0.001 to 1 mole of tin (II) nitrate (V) per 1 kg of bentonite. More preferably, the following proportions of bentonite and metal salts can be used: 0.005 mole of silver (I) nitrate (V) per 1 kg of bentonite, 0.005 mole of zinc (II) nitrate (V) per 1 kg of bentonite, 0.005 mole of copper (II) nitrate (V) per 1 kg of bentonite, and 0.01 to 1 mole of other metal Me (II) nitrate (V) per 1 kg of bentonite, including, for example, tin (II) nitrate (V), zirconium (II) nitrate (V), nickel (II) nitrate (V) or cobalt (II) nitrate (V). In the case of such selection of amounts of reaction reagents, regardless of the type of various metal ions used, for example: two different ions, or more than two different ions, all the metal ions, introduced into the solution, are fully intercalated in the bentonite grains, which additionally increases the raw material efficiency of the process and reduces the amount of generated wastewater.

The above values are selected independently of the amount of the various metal ions. In particular, in step 103, to the aqueous suspension of bentonite can be introduced, in the form of respective metal nitrates, at least two different metal ions, or, for example three different metal ions, or four different metal ions selected from the group consisting of: Ag⁺, Cu²⁺, Zn²⁺, Sn²⁺, Ni²⁺, Zr²⁺, Co²⁺ and Bi²⁺ in an appropriate concentration and in any combination thereof.

After all the nitrates have been introduced into the aqueous bentonite suspension, mixing is preferably carried out for the time necessary for the substitution, in the bentonite exchange positions, of all metal ions introduced in ionic form: water-soluble metal nitrogen (V) salts, which is preferably from 1 hour to 3 hours depending on the mass of bentonite. The reaction temperature is kept in the range of 10 to 30° C while mixing.

As a result of mixing, in step 103, the following product is obtained: a modified bentonite 10 in the form of an aqueous suspension, which has at least two different metal ions intercalated in each individual grain of bentonite. The obtained product shows an improved biostatic effect against, among others, pathogens such as: *Enterobacteriaceae, Escherichia coli, Rhodotorula mucilaginosa,* and a biocidal effect against, among others, pathogens such as: *Serratia marcescens, Bacillus subtilis, Bacillus megatherium.* The obtained aqueous suspension of modified bentonite 10 may optionally be mixed with the addition of a modified zeolite preferably containing the same metal ions as the modified bentonite, and thus in the form of a biocidal composition, and may be diluted with water to obtain a modified bentonite concentration in this suspension preferably in the range of 10 to 50 wt.%. The manufactured aqueous suspension with the possible addition of zeolite can be used in the manufacturing of various products to give them biocidal properties, such as cleaning agents, cosmetics, building materials, or cellulose materials, for example paper or cardboard, by spraying it on selected surfaces of these products, or introducing as a mass component at the product manufacturing stage. Furthermore, the modified bentonite 10 can be separated from the suspension, for example by filtration, and used as a loose biocidal additive for various products.

Optionally, in step 104, the obtained aqueous suspension of modified bentonite may be further thickened and washed with water or chelate in order to remove nitrate ions therefrom. Then, in step 105, the whole may be dewatered, for example to a dryness degree in the range of 20 to 80 wt.%, thereby yielding a purified and partially dried product. The advantage of thickening and washing in step 104 is to reduce the amount of nitrate ions in the product and to reduce the amount of water that is transported with the product.

Optionally, the dewatered modified bentonite may be mixed in step 107 with the previously prepared mixture of cellulose nano-fibres and cationic polyelectrolyte, manufactured in step 106. The manufactured composition of modified bentonite with cellulose fibres and cationic polyelectrolyte 20 shows improved adhesion to various surfaces, therefore it is particularly suitable for spray application on various types of products in order to give them biostatic and biocidal properties against various pathogens. Optionally, an ion-exchanged zeolite can be introduced into the mixture in step 107. The addition of zeolite in any case, regardless of the form of modified bentonite, including, for example, an aqueous suspension or a loose mixture, while preferably maintaining the weight proportions: modified bentonite : zeolite: 50:1 to 5:1 provides additional properties of the product: including the increase in the biocidal effect, reduction the viscosity of the resulting suspension.

The obtained product: modified bentonite 10 and various compositions containing the modified bentonite, including an aqueous composition with optional cellulose fibres 20 and/or addition of zeolite, preferably containing the same metal ions as the intercalated bentonite grains, can be used as a biocidal and biostatic additive for, among others, paper, cardboard, as well as other cellulose products, toilet and hygiene utensils, for example diapers or paper towels, or construction materials, including wood-like panels, bricks, honeycomb panels, used for, among others, manufacturing of furniture and interior woodwork, as well as an additive to cleaning agents, including, for example: soaps, creams, washing liquids, or dressing materials, ointments, gels, including for use on wounds. The modified bentonite and the above compositions containing the modified bentonite may be introduced into products in various ways in order to impart biostatic and biocidal properties, for example by spraying methods or as an ingredient of the product. In the case of manufacturing dressings, compositions containing the modified bentonite can be sprayed onto the dressing or used as an additive to the material the dressing is made of.

The modified bentonite, as well as various compositions comprising the modified bentonite, can be applied on cellulosic materials such as cardboard or paper by spraying, or they can be added to the paper pulp as an ingredient thereof. The spraying method is more effective because the entire amount of the modified bentonite is applied to the treated surface. Using the developed method, an aqueous suspension of the modified bentonite or its various compositions, including the ones with the addition of zeolites and or cellulose nano-fibres, preferably with a concentration of modified bentonite of at least 0.2%, can be sprayed onto the surface. For example, for the manufacturing of paper, the spraying of a composition with the modified bentonite may be carried out by means of wire or in the press section of the papermaking machine, most preferably in an amount necessary to obtain a surface coverage with the modified bentonite in the range of 0.05 to 5 g/m², the optimal amount of the modified bentonite dosed being selected according to the specific needs, including the type of paper and the degree of biostatic and biocidal properties required.

In the case of building materials, the modified bentonite or compositions containing thereof can be sprayed onto selected surfaces, or they can be added directly to the mass at the stage of manufacturing these products. However, as an additive to cosmetics, the modified bentonite or compositions containing thereof can be added as an ingredient of said products.

For example, the modified bentonite may be provided in a form of paste constituting the modified bentonite and liquid composition, such as the modified bentonite and water slurry, for example of substantially high viscosity. The paste may be prepared by mixing activated bentonite powder with a selected liquid, preferably water to obtain a slurry, at a desired stirring speed, preferably of 0,2 to 2,0 rps (revolution per second), and more preferably of 0,5 rps, for 30 to 120 minutes. The stirring speed over 2,0 rps might negatively affect the rheological properties of the obtained paste, i.e. shear stresses in the slurry system might become exceeded and the system might lose its elastic component and become a typical viscous suspension (but not of paste consistency).

Next, the slurry is fed with at least two different water solutions of metal nitrates, and the stirring is continued till obtaining the desired hetero-intercalation level of bentonite grains - preferably all the introduced metal ions are substituted within the bentonite. Distilled or demineralized water is preferred to be used, as the presence of impurities such as manganese and/or iron cations may considerably reduce the biocidal properties of hetero-intercalated bentonite. Further, the reduction of bentonite powder concentration in the tank, during the stirring with respective metal nintrates, below 4% (based on the dry mass of bentonite), may additionally reduce the biocidal properties of the obtained hetero-intercalated bentonite. However, once a paste of the modified bentonite is obtained, it can be further diluted to a desired consistency and concentration of the modified bentonite. Also, it is preferred to maintain the temperature in the tank, not higher than 50°C, as higher temperature in the system may increase the risk of silver precipitation, thereby, disrupting even level of bentonite grains hetero-intercalation with different metal ions. After the stirring is completed, the product in the tank is of paste (viscous slurry) consistency and it is ready-to-use.

The paste may be applied to the various substrates to impart their biostatic and biocidal properties. The past with the modified bentonite, or another composition comprising the modified bentonite, according to the present disclosure, may be applied to surfaces in various ways. For example, the past prepared as described above, comprising, e.g., 10% of the modified bentonite may be applied using roller on the desired surface (e.g. Roller Coating Process), and the paste excess may be collected with Meyer's rod (a formed rod that leaves a certain amount of paste on the substrate). This coating method may be employed to cellulose substrates such as papers and cardboards of grammage not less than 70g/m².

Spraying is another exemplary method for the application of the modified bentonite on various substrates to impart their biostatic and biocidal properties. For example, the modified bentonite in a form of suspension may be sprayed on substrates such as sanitary-hygienic tissue papers called also tissues, typically made from cellulosic materials of grammage not higher than 40g/m², and typically of grammage of 25g/m². Contrary to the rodcoating method, the spraying method may be employed for cellulose substrates such papers of lower grammages, even at the level of 1g/m².

In order to verify the synergistic properties of the modified bentonite in which a single grain is intercalated with ions of at least two different metals, the following microbiological tests were carried out on paper from recycled pulp containing the modified bentonite. Recycled paper which, by its nature, contains a large amount of microbiological contamination was used for the tests. The bentonite was applied by spraying in an amount of about 0.25 g/m². Table 1 below summarises the characteristics of the tested samples and the results of the microbiological test - content measurements: *Enterobacteriaceae,* the test was carried out by means of swabbing from the surface of the product using ready-made swabs with peptide buffer. Then, the buffer samples were applied to disposable ready-made media and incubated for 24 hours at 35°C.

**Table 1 - the results of measurements of the Enterobacteriaceae content in the samples:**

| **Addition of ions in a single grain of bentonite** | **Sample** | **Amount of bacteria in the sample** |
|---|---|---|
| None (reference) | paper from recycled pulp without the addition of bentonite | 1800 +/- 340 cfu/g |
| Grain contains: silver (Ag⁺) | paper from recycled pulp with the addition of bentonite (content: Ag 0.02 mol/kg of bentonite) | 380 +/-70 cfu/g |
| Grain contains: silver (Ag⁺) | paper from recycled pulp with the addition of bentonite (Ag 0.1 mol/kg) | 160 +/-45 cfu/g |
| Grain contains: zinc (Zn²⁺) | paper from recycled pulp with the addition of bentonite (Zn 0.4 mol/kg): | 880 +/-220 cfu/g |
| Mixture: grains with Ag⁺ and grains with Zn²⁺, in a weight ratio of 50:50 | paper from recycled pulp with the addition of bentonite, 50% (Ag 0.02 mol/kg) and 50% (Zn 0.4 mol/kg): | 86 +/-48 cfu/g |
| Grain contains: silver + zinc (Ag⁺, Zn²⁺) | paper from recycled pulp with the addition of bentonite (Ag 0.02 mol/kg and Zn 0.4 mol/kg): | 42 +/-22 cfu/g |
| Grain contains: silver + copper (Ag⁺, Cu²⁺) | paper from recycled pulp with the addition of bentonite (Ag 0.02 mol/kg and Cu 0.4 mol/kg): | 68 +/-28 cfu/g |

Table 2 summarises, in turn, the results of obtained measurements of microbiological activity of the modified bentonite against fungi and moulds. Testing methodology: samples were collected with a swab immersed in buffer. Then, 1ml or its dilution was transferred to disposable ready-made media: 3M Food Safety's (Saint Paul, USA) *3M*^{™} *Petrifilm*^{™} *Yeast and Mold Count Plates,* which allow mould and fungus testing in general, and incubated for 4 days at 25°C.

**Table 2 - the results of measurements of mould and fungus content in the samples:**

| **Addition of ions in a single grain of bentonite** | **Sample** | **Amount of mould/fungus in the sample** |
|---|---|---|
| None (reference) | paper from recycled pulp without the addition of bentonite | 2400 +/- 700 cfu/g |
| Grain contains: silver (Ag⁺) | paper from recycled pulp with the addition of bentonite (content: Ag 0,02 mol/kg of bentonite) | 600 +/-200 cfu/g |
| Grain contains: zinc (Zn²⁺) | paper from recycled pulp with the addition of bentonite (Zn 0.4 mol/kg): | 1200 +/-200 cfu/g |
| Grain contains: copper (Cu²⁺) | paper from recycled pulp with the addition of bentonite (Cu 0.4 mol/kg): | 400 +/-100 cfu/g |
| Grain contains: silver + zinc (Ag⁺, Zn²⁺) | paper from recycled pulp with the addition of bentonite (Ag 0.02 mol/kg and Zn 0.4 mol/kg): | 400 +/-100 cfu/g |
| Grain contains: silver + copper (Ag⁺, Cu²⁺) | paper from recycled pulp with the addition of bentonite (Ag 0.02 mol/kg and Cu 0.4 mol/kg): | 120 +/-60 cfu/g |

The results of the conducted tests confirmed the synergistic effect of two different cations contained in a single grain of the modified bentonite according to the developed method. Using bentonite intercalated with silver alone, even in the maximum dose which amounts to Ag 0.1 mole per 1 kg of bentonite - and which therefore theoretically (stoichiometrically) allows all silver ions to be included in the bentonite structure, and thereby without process losses - the obtained biocidal effectiveness was smaller than in the case of the system of two cations: silver and zinc as well as silver and copper intercalated in a single grain of bentonite. It should therefore be noted that in the case of the sample obtained with the solution of silver ions: Ag 0.1 mole per 1 kg of bentonite - bentonite with the maximum possible amount of Ag ions contained in the bentonite structure is obtained, and its activity against *Enterobacteriaceae* is significantly lower - by an order of magnitude for the result expressed in [cfu/g], compared to bentonite intercalated with ions of two different metals in the grain. Furthermore, the mixture of two different bentonite powders containing grains only with Ag⁺ and grains only with Zn²⁺ showed biocidal properties half as good compared to the modified bentonite containing the same ions but contained in a single bentonite grain, which additionally confirmed the synergistic properties of the maintained system.

Based on the tests, it can also be concluded that the modified bentonite containing silver and copper ions in a single grain has a beneficial effect on fungi and moulds.

Therefore, the conducted test confirmed the synergistic effect of the modified bentonite manufactured by the developed method. In particular, the use of a five times lower dose of silver and the addition of copper or zinc in a single grain of bentonite allowed the amount of microorganisms in the samples to be reduced by an order of magnitude, compared to grains with one type of intercalated metal ion.

Further, a product in a form of a paste comprising 10% of the modified bentonite having single grains intercalated with ions of at least two different metals, was tested to determine its bactericidal activity against Escherichia coli K12 NCTC 10538 according to ISO 13727 - the obtained results for different samples are set in Table 3, below.

The tests were carried out at bovine albumin 0.3 g/l, (for each sample the paste sample was mixed with E.coli inoculum, and next rubbed ); test temperature: 20°C; for each sample, the test was carried out for 60 s, the samples to be tested were incubated at 37°C. The concentrations during the test were as follows: 97% of the paste comprising 10% of respective bentonite powder (as described in Table 3), 3% of additives (water and serum albumin), the tested material was not diluted.

Sample preparation: all the pastes were prepared on the same test stand, for batches of 600 g of dry bentonite, the final paste concentrations were 10% of modified bentonite. The volumes of respective metal nitrogen salts feed to the bentonite powder, at the modification of each bentonite sample, were at the same level of 500 ml for each metal nitrate water solution used (different mole concentrations of the respective water solutions of metal nitrates), as set in Table 3. Feeding duration of metal nitrate water solution: 20 minutes, each sample.

Detailed preparation of samples: dry, activated bentonite was sieved on the sieve on mesh no 400 (nominal sieve opening - 37 microns). Next, the bentonite was weighed, and 600 g of bentonite powder was added at once (as one portion to prepare individual paste sample) to a stirring tank with a thermostatic jacket (the heating/cooling jacket) following the introduction of 5 liters of distilled water of conductance less than 5 microsiemens. During all the processes for pastes preparation, the temperature measured in the tank was maintained at the level of about 25°C. The water with bentonite powder was stirred at 0,5 rps (revolution per second), and a highly viscous paste of concentration 12% was obtained in the tank. The stirring was carried out for 30 to 60 minutes till a substantially homogenous suspension was obtained, without visible clumps of unwet bentonite powder. Next, respective solution(s) of metal nitrate, e.g., silver nitrate and copper nitrate were introduced (fed) into the tank with a peristaltic pump, at a flow rate of 0,4 ml/s - different solutions of metal nitrates for each sample, detailed concentrations and ratios are set in Table 3. For the bentonite hetero-intercalated, according to the present disclosure (Table 3, Test samples - with the modified bentonite), two respective water solutions of different metal nitrates were simultaneously feed with peristaltic pumps at a flow fate of 0,4 ml/s, into the tank with the continuously stirred water-bentonite suspension. The feeding system was configured so that the respective two different metal cations were introduced into the tank at a certain distance from each other, and the stirring process was affected efficiently to quickly mix the bentonite suspension with the metal nitrates.

For all the prepared samples, after all the amounts of respective metal nitrate(s) were added into the tank, the suspension in the tank was continuously stirred for about 30 minutes. The used stirring system provided good primary and secondary circulation of the stirred tank content.

The obtained pastes were viscous suspensions of 10% concentration of bentonite (based on bentonite dry mass). The obtained pastes, in the same amount for each sample, were mixed with E.coli inoculum and next rubbed..

**Table 3 - pates comprising 10% of bentonite, the bactericidal activity of the samples against Escherichia coli K12 NCTC 10538 according to ISO 13727**

| **Addition of ions in a single grain of bentonite** | **Sample description** | **Bacterial reduction (log) IgN₀-IgN_{A}** | **Sample type** |
|---|---|---|---|
| None (reference) | Paste with 10% of bentonite (unmodified) | 0,59 | Blank |
| Grain contains silver (Ag⁺) dose no 1 | Paste with 10% of bentonite (modified with Ag - 0,02 mol/kg, i.e., 3,38 g/kg of bentonite) | 2,28 | Reference |
| Grain contains silver (Ag⁺) dose no 1 | Paste with 10% of bentonite (modified with Ag - 0,04 mol/kg, i.e., 6,76 g/kg of bentonite) | 4,20 | Reference |
| Grain contains silver (Ag⁺) dose no 3 | Paste with 10% of bentonite (modified with Ag - 0,08 mol/kg, i.e., 13,52 g/kg of bentonite) | 4,56 | Reference |
| Grain contains zinc (Zn²⁺) dose no 1 | Paste with 10% of bentonite (modified with Zn 0,2 mol/kg, i.e., 37,87 g/kg of bentonite): | 1,50 | Reference |
| Grain contains zinc (Zn²⁺) dose no 2 | Paste with 10% of bentonite (Zn 0,4 mol/kg, i.e., 75,74 g/kg of bentonite): | 2,22 | Reference |
| Mixture: grain with Ag⁺ and grain with Zn²⁺, in a weight ratio 50: 50, dose no 1 | Mixture of two pastes, each paste comprising 10% of bentonite, one paste with bentonite modified with silver ions only (Ag 0,04 mol/kg), and the other paste with bentonite modified with zinc ions only (Zn 0,4 mol/kg), the pastes are mixed in a weight ratio of 1: 1; the average amount of Ag and Zn in the mixture is of 0,02 of Ag and 0,2 of zinc [mol per kg of bentonite] | 2,86 | Comparable |
| Grain contains: silver + zinc (Ag⁺, Zn²⁺) dose no 1 | Paste with 10% of bentonite hetero-intercalated, each grain with Ag and Zn ions (Ag 0,02 mol/kg and Zn 0,2 mol/kg) | 4,69 | **Test (with the modified bentonite)** |
| Mixture: grain with Ag⁺ and grain with Zn²⁺, in a weight ratio 50: 50, dose no 2 | Mixture of two pastes, each paste comprising 10% of bentonite, one paste with bentonite modified with silver ions only (Ag 0,08 mol/kg) and the other paste with bentonite modified with zinc ions only (Zn 0,8 mol/kg), the pastes are mixed in a weight ratio of 1: 1; the average amount of Ag and Zn in the mixture is of 0,04 of Ag and 0,4 of Zn [mol/kg] | 5,06 | Comparable |
| Grain contains: silver + zinc (Ag⁺, Zn²⁺) dose no 2 | Paste with 10% of bentonite hetero-intercalated, each grain with Ag and Zn ions (Ag 0,04 mol/kg and Zn 0,4 mol/kg) | 5,92 | **Test (with the modified bentonite)** |
| Grain contains: copper (Cu²⁺) dose no 2 | Paste with 10% of bentonite (modified with Cu 0,4 mol/kg, i.e., 75,02 g/kg of bentonite): | 1,32 | reference |
| Mixture: grain with Ag⁺ and grain with Cu²⁺, in a weight ratio 50:50, dose no 2 | Mixture of two pastes, each paste comprising 10% of bentonite, one paste with bentonite modified with silver ions only (Ag 0,08 mol/kg) and the other paste with bentonite modified with copper ions only (Cu 0,8 mol/kg), the pastes are mixed in a weight ratio of 1: 1; the average amount of Ag and Cu in the mixture is of 0,04 of Ag and 0,4 of Cu [mol/kg] | 3,89 | Comparable |
| Grain contains: silver + copper (Ag⁺, Cu²⁺), dose no 2 | Paste with 10% of bentonite (modified with Ag 0,04 mol/kg and Cu 0,4 mol/kg) | 4,06 | **Test (with the modified bentonite)** |
| Grain contains: nickel (Ni²⁺) dose 1 | Paste with 10% of bentonite (Ni 0,1 mol/kg, i.e., 18,27 g/kg bentonitu) | 2,51 | reference |
| Grain contains: nickel I (Ni²⁺) dose 2 | Paste with 10% of bentonite (Ni 0,2 mol/kg, i.e., 36,54 g/kg of bentonite) | 3,05 | reference |
| Mixture: grain with Ag⁺ and grain with Ni²⁺, in a weight ratio 50: 50, dose 1 | Mixture of two pastes, each paste comprising 10% of bentonite, one paste with bentonite modified with silver ions only (Ag 0,04 mol/kg) and the other paste with bentonite modified with nickel ions only (Ni 0,2 mol/kg), the pastes are mixed in a weight ratio of 1: 1; the average amount of Ag and Ni in the mixture is of 0,02 of Ag and 0,1 of Ni [mol/kg] | 4,51 | Comparable |
| Grain contains: silver + copper (Ag⁺, Ni²⁺) dose no 1 | Paste with 10% of bentonite (modified with Ag 0,02 mol/kg and Ni 0,1 mol/kg) | 4,89 | **Test (with the modified bentonite)** |
| Mixture: grain with Ag⁺ and grain with Ni²⁺, in a weight ratio 50: 50 dose 2 | Mixture of two pastes, each paste comprising 10% of bentonite, one paste with bentonite modified with silver ions only (Ag 0,08 mol/kg) and the other paste with bentonite modified with nickel ions only (Ni 0,4 mol/kg), the pastes are mixed in a weight ratio of 1: 1; the average amount of Ag and Ni in the mixture is of 0,04 of Ag and 0,2 Ni [mol/kg] | 5,69 | Comparable |
| Grain contains: silver + copper (Ag⁺, Ni²⁺) dose no 2 | Paste with 10% of bentonite (modified with Ag 0,04 mol/kg and Ni 0,2 mol/kg) | 6,47 | **Test (with the modified bentonite)** |

As follows from Table 3, the samples of pastes comprising hetero-intercalated bentonite, i.e., with different metal cations comprised in an individual bentonite grain (test samples with the modified bentonite), show increased bactericidal activity against Escherichia coli - bacteria reduction even higher than 1 order of magnitude, compared with the samples of mixed bentonite powders (comparable samples, with the known mixtures of bentonite powders). This demonstrates improved antibacterial properties of the modified bentonite according to the present disclosure. In detail, the antibacterial activity against E. Coli is stronger for the silver-nickel hetero-intercalated bentonite grains and strong for the silver-zinc hetero-intercalated bentonite grains. As follows from Table 3, the silver-nickel hetero-intercalated bentonite grains, even at lower ionic concentrations than those for bentonite with zinc or copper, show substantially high antibacterial effect. The hetero-intercalated bentonite grains comprising silver and copper shown a slightly weaker antibacterial effect. This is however consistent with the literature data, as copper is known to exhibit rather antifungal activity.

Further, for the paste with the modified bentonite having bentonite grains (hetero-intercalated) with Ag and Zn ions (where the bentonite water slurry was fed with salts solutions: 0,016 mol AgNOs and 0,016 mol Zn(NO₃)₂ per 1 kg bentonite powder), a test according to standard: EN13727+A2:2015-12 was conducted at a bovine albumin loading of 0,3 g/l, duration time 60s, at 20°C. Tested sample: 97% of the paste comprising 10% of the modified bentonite, 3% of additives (water and serum albumin). The obtained result bacterial reduction: IgN₀-IgN_{A} (Escherichia coli K12 NCTC 10538) was 5,34, which confirmed improved antibacterial properties of the modified bentonite.

Further, cellulose substrates with a paste comprising 8% of the modified bentonite were tested against Escherichia coli K12 NCTC 10538 according to ISO 20743 - the obtained results for different samples are set in Table 4 below.

**Table 4 - pastes comprising 8% of bentonite on a cellulose substrate, the bactericidal activity of the samples against Escherichia coli K12 NCTC 10538 according to ISO 20743.**

| **Addition of ions in a single grain of bentonite** | **Sample description** | **Bacterial reduction IgN₀-IgN_{A}** | **Sample type** |
|---|---|---|---|
| Mixture: grain with Ag⁺ and grain with Zn²⁺, in a weight ratio 50: 50, dose no 1 | Mixture of two pastes, each paste comprising 10% of bentonite, one paste with bentonite modified with silver ions only (Ag 0,08 mol/kg) and the other paste with bentonite modified with zinc ions only (Zn 0,4 mol/kg), the pastes are mixed in a weight ratio of 1: 1; the average amount of Ag and Zn in the mixture is of 0,02 of Ag i 0,2 of Zn [mol/kg] | 5,69 | Comparable |
| Grain contains: silver + zinc (Ag⁺, Zn²⁺) dose no 1 | Paste with 10% of bentonite (modified with Ag 0,02 mol/kg and Zn 0,2 mol/kg) | 6,48 | **Test (with the modified bentonite)** |
| Mixture: grain with Ag⁺ and grain with | Mixture of two pastes, each paste comprising 10% of bentonite, one paste with bentonite modified with silver ions | 6,12 | Comparable |
| Zn²⁺, in a weight ratio 50: 50, dose no 2 | only (Ag 0,08 mol/kg) and the other paste with bentonite modified with zinc ions only (Zn 0,8 mol/kg), the pastes are mixed in a weight ratio of 1: 1; the average amount of Ag and Zn in the mixture is of 0,04 of Ag and 0,4 of Zn [mol/kg] | | |
| Grain contains: silver + zinc (Ag⁺, Zn²⁺) dose no 2 | Paste with 10% of bentonite (modified with Ag 0,04 mol/kg and Zn 0,4 mol/kg), | 6,62 | **Test (with the modified bentonite)** |

As follows from Table 4, the samples of cellulosic substrates coated with pastes comprising of 8% hetero-intercalated bentonite, i.e., where each bentonite grain comprises two different ions (test samples with the modified bentonite), show increased bactericidal activity against Escherichia coli, i.e., bacteria reduction was higher of about 0,5 order of magnitude, compared with the samples of mixed bentonite powders (comparable samples, with the known mixtures of bentonite powders), which further demonstrates improved antibacterial properties of the modified bentonite according to the present disclosure, thereby, further confirming the increased bactericidal activity of the modified bentonite according to the present disclosure.

### EMBODIMENT 1 - modified bentonite applied on paper by spraying

A portion of the activated bentonite containing sodium and potassium in its structure (Na/K-bentonite) was sieved through a sieve 400, wherein the mesh size was below 33 microns. The fines from the sieve with a mass of 1 kg were obtained and were directed to further processes, including mixing bentonite with 3 litres of demineralised water to obtain a suspension of bentonite with a bentonite content of about 35%. In turn, separately, in 0.5 dm³ of demineralised water, 3.4 g (i.e. 0.02 mole) of silver nitrate (AgNOs) was prepared. In a separate tank, in 0.5 dm³ of demineralised water, 178 g of Zn(NO₃)₂•6H₂O (i.e. 0.6 moles) was dissolved. The bentonite suspension was then transferred to a mixer where it was subjected to continuous mixing. The mixer was a cylindrical tank equipped with a stirrer and a heating/cooling jacket. Silver nitrate was added to the mixer in small portions so as to obtain the best possible distribution of the ingredient throughout the volume. The time of adding the silver nitrate was about 20 minutes, i.e. the average volumetric flow of the silver nitrate solution was about 0.8 ml/s (i.e.: 50 ml/min). Zinc nitrate was then added in an analogous manner. The time of adding the zinc nitrate was also 20 minutes. During the addition of both ingredients, the bentonite was constantly mixed in such a way that the content of the mixer was in constant motion and it did not allow local saturation of the bentonite with ions of a given metal. After the addition of the ingredients, the bentonite was further mixed for 3 hours in order to obtain a uniform concentration of silver and zinc ions throughout the volume. During mixing, the temperature of the bentonite was kept at the level of 20-30°C by means of the water jacket.

The obtained modified bentonite suspension - without dewatering or further treatment, was a finished product which, after dilution to a bentonite concentration of 0.5%, can be applied, for example, by spraying directly to various surfaces, including cellulose surfaces such as paper.

### Microbiological testing:

As part of microbiological testing, the modified bentonite obtained according to Example 1 was applied to paper obtained from recycled pulp and to paper obtained from cellulose pulp, then samples were prepared from each type of paper in such a way that a swab was taken from the surface of the paper using ready-made swabs with a peptide buffer, and buffer samples were applied to a disposable medium which was incubated at 35°C for: 24h. Then, the *Enterobacteriaceae* content was tested by counting the colonies, and then the results were given in the units of cfu/g and summarised below:
The test was performed using ready-made swab plates:
- for the samples of paper from cellulose pulp without the addition of bentonite (reference): 440 +/- 50 cfu/g,
- for the samples of paper from recycled pulp without the addition of bentonite (reference): 1000 +/- 100 cfu/g,
- for the samples of paper from cellulose pulp with the addition of modified bentonite: 35 +/-16 cfu/g,
- for the samples of paper from recycled pulp with the addition of modified bentonite: 60 +/-28 cfu/g.

Thus, microbiological testing confirmed very good antibacterial properties of cellulose pulps with a layer of modified bentonite applied to the surface of these products by spraying.

### EMBODIMENT 2 - composition based on modified bentonite, containing cellulose nano-fibres and cationic polyelectrolyte

A portion of the activated bentonite containing sodium and potassium in its structure (Na/K-bentonite) was sieved through a sieve 400, with a mesh size below 33 microns. The bentonite fines with a mass of 1 kg were obtained and were directed to further processes, including mixing bentonite with 4 litres of demineralised water to obtain a suspension of bentonite with a bentonite content of about 25%. In turn, separately, in 0.5 dm³ of demineralised water, 5.1 g (i.e. 0.03 mole) of silver nitrate (AgNOs) was prepared. In a separate tank, in 0.5 dm³ of water, 148 g (i.e. 0.5 mole) of copper nitrate: Cu(NO₃)₂•6H₂O was dissolved. The bentonite suspension was then transferred to a mixer, where the whole was subjected to continuous mixing process. The mixer was a cylindrical tank equipped with a stirrer and a heating/cooling jacket. Silver nitrate was added to the mixer in small portions so as to obtain the best possible distribution of the ingredient throughout the mass. The time of adding the silver nitrate was about 10 minutes, i.e. the average volumetric flow of the silver nitrate solution was about 0.8 ml/s (i.e.: 50 ml/min). Copper nitrate was then added in an analogous manner. The time of adding the copper nitrate was also 10 minutes. During the addition of both ingredients, the bentonite was constantly mixed in such a way that the content of the mixer was in constant motion and it did not allow local saturation of the bentonite with ions of a given metal. After the addition of the ingredients, the bentonite was further mixed for 3 hours in order to obtain a uniform concentration of silver and copper ions throughout the volume. During mixing, the temperature of the bentonite was kept at the level of 20-30°C by means of the water jacket.

Then, the obtained bentonite suspension was diluted with demineralised water to obtain a concentration of: 10%, i.e. additional 5 dm³ of water was added. Thus, the total volume of the suspension was about 10 dm³. The dilution process was carried out in the same mixer, where the ion exchange (i.e. the intercalation of silver and copper ions) occurred. After about 15 minutes of mixing, the content of the mixer was transferred to the dewatering system in the form of a nutsche filter and excess water was drawn off until the suspension had a dryness of about 25%. The concentrated suspension was returned to the mixer and washing was repeated 3 times. During this process, the content of nitrates in the suspension decreased from about 60 grams to about 10 grams. The separated filtrate containing sodium and potassium nitrates could be used as a substitute for demineralised water in the process of preparing a new portion of bentonite suspension.

Then, in a separate tank, 50 g of cellulose nano-fibres with an average size in the range of 50 nm - 500 nm was prepared and diluted in 1 dm³ of water. Then, about 2 ml of polyacrylamide solution with a concentration of 0.25% - as an addition of cationic polyelectrolyte - was then added to the obtained suspension. The whole was mixed without excessive aeration. The addition of this polyelectrolyte allowed a neutral electrokinetic potential to be obtained, i.e. the electrokinetic potential being close to 0 mV

Then, the obtained bentonite and cellulose nano-fibres suspension with bentonite was mixed, gradually adding cellulose nano-fibres and polyelectrolyte to the bentonite. The whole process was carried out by mixing the whole with very limited turbulence. The mixing time was 15 minutes, while the temperature during mixing had to be below 35°C. The recommended temperature of the process was 20 to 30°C.

Then, the obtained composition of the modified bentonite, cellulose nano-fibres and cationic polyelectrolyte (polyacrylamide) without dewatering or further treatment thereof was used as a finished product which, after diluting with water to obtain a modified bentonite concentration of 5%, was dosed to the papermaking machine in order to manufacture paper with biocidal and biostatic properties.

### EMBODIMENT 3 - modified bentonite containing three different metal ions in a single bentonite grain

Activated bentonite containing sodium and potassium in its structure (Na/K-bentonite) was sieved through a sieve 400, with a mesh size below 33 microns. The obtained bentonite fines in an amount of 1 kg were directed to a further process in which the bentonite fines were mixed with 4 litres of demineralised water to obtain a suspension with a bentonite content of about 25%. In turn, separately, in 0.5 dm³ of demineralised water, 3.4 g (i.e. 0.02 mole) of silver nitrate (AgNOs) was prepared. In a separate tank, in 0.5 dm³ of water, 59.4g (i.e. 0.2 mole) of zinc nitrate: Zn(NO₃)₂•6H₂O was dissolved. In another tank, 0.5 dm³ of water was prepared, where 59.2 g (i.e. 0.2 mole) of copper nitrate: Cu(NO₃)₂•6H₂O was dissolved. The bentonite suspension was then transferred to a mixer, where the bentonite was subjected to continuous mixing process. The mixer was a cylindrical tank equipped with a stirrer and a heating/cooling jacket. Silver nitrate was added to the mixer in small portions so as to obtain the best possible distribution of the ingredient throughout the mass. The time of adding the silver nitrate was about 10 minutes, i.e. the average volumetric flow of the silver nitrate solution was about 0.8 ml/s (i.e.: 50 ml/min). Zinc nitrate and copper nitrate were then added in an analogous manner. The time of adding the zinc nitrate was also 10 minutes. The time of adding the copper nitrate was also 10 minutes. The order of the dosed nitrates was as follows: silver, zinc and copper. During the addition of all three ingredients, the bentonite was constantly mixed in such a way that the content of the mixer was in constant motion and it did not allow local saturation of the bentonite with ions of a given metal. After the addition of all the nitrates, the bentonite was further mixed for 3 hours in order to obtain a uniform concentration of silver, zinc and copper ions throughout the volume. During mixing, the temperature of the bentonite was kept at the level of 20-30°C by means of the water jacket.

Then, the obtained bentonite without dewatering or further treatment was the finished product which, after dilution to a concentration of 0.5%, could be sprayed directly on construction materials. Thus, a modified bentonite was obtained in which each single grain contained intercalated three different metal ions: Ag⁺, Cu²⁺ and Zn²⁺.

### EMBODIMENT 4 - composition based on modified bentonite, containing cellulose nano-fibres and cationic polyelectrolyte for spray application on dressings

A portion of the activated bentonite containing sodium and potassium in its structure (Na/K-bentonite) was sieved through a sieve 400, with a mesh size below 33 microns. The fines with a mass of 1 kg were obtained from the sieve and were directed to a further process in which 1 kg of bentonite was mixed with 4 litres of demineralised water to obtain a suspension with a bentonite content of about 25%. In turn, separately, in 0.5 dm³ of demineralised water, 8.5 g (i.e. 0.05 mole) of silver nitrate (AgNOs) was prepared. In a separate tank, in 0.5 dm³ of water, 148 g (i.e. 0.5 mole) of copper nitrate: Cu(NO₃)₂•6H₂O was dissolved. The bentonite suspension was then transferred to a mixer, where the whole was subjected to continuous mixing process. The mixer was a cylindrical tank equipped with a stirrer and a heating/cooling jacket. Silver nitrate was added to the mixer in small portions so as to obtain the best possible distribution of the ingredient throughout the mass. The time of adding the silver nitrate was about 10 minutes, i.e. the average volumetric flow of the silver nitrate solution was about 0.8 ml/s (50 ml/min). Copper nitrate was then added in an analogous manner. The time of adding the copper nitrate was also 10 minutes. During the addition of both ingredients, the bentonite was constantly mixed in such a way that the content of the mixer was in constant motion and it did not allow local saturation of the bentonite with ions of a given metal. After the addition of the ingredients, the bentonite was further mixed for 3 hours in order to obtain a uniform concentration of silver and copper ions throughout the volume. During mixing, the temperature of the bentonite was kept at the level of 20-30°C by means of the water jacket.

Then, the obtained suspension of modified bentonite was diluted with demineralised water to a modified bentonite concentration of 5%, i.e. additional 15 litres of water were added. The total volume of the suspension was about 20 dm³. The dilution process was carried out in the same mixer, where the ion exchange occurred. After about 15 minutes of mixing, the content of the mixer was transferred to the dewatering system in the form of a centrifuge and excess water was drawn off until the suspension had a dryness of about 35%. The concentrated suspension was returned to the mixer and the process of washing was repeated 5 times. As a result of washing, the content of nitrates in the suspension decreased from about 62 grams to below 3 grams. The separated filtrate containing sodium and potassium nitrates could be used as a substitute for demineralised water in the process of preparing aanother portion of bentonite suspension.

Then, in a separate tank, 200g of cellulose nano-fibres with an average size of 250 nm was prepared and diluted in 1 dm³ of water. Then, 10 g of cationic starch (as a cationic polyelectrolyte) was added to the suspension and the whole was heated to 95°C. This temperature was held for 5 minutes while continuously mixing. The suspension was then cooled to a temperature below 30°C.

Then, the obtained bentonite suspension was mixed with a suspension of cellulose nano-fibres with cationic starch, gradually introducing the suspension of cellulose nano-fibres and cationic starch into the bentonite suspension. The process was carried out while mixing the whole under high turbulence conditions: with a high rotational speed of the stirrer, exceeding 2000 rpm, to obtain a homogeneous suspension. The mixing speed was then reduced and the mixing process was carried out under reduced turbulence for another 10 minutes. The temperature during mixing was between 20 and 30°C.

The thus obtained aqueous composition of modified bentonite, cellulose nano-fibres and cationic polyelectrolyte, without dewatering or further treating thereof, was used as a finished product which, after dilution with water to obtain a modified bentonite concentration of 2%, can be sprayed onto the surface of tissue papers made of cellulose nano-fibres, for use as a dressing material.

### EMBODIMENT 5 - composition based on modified bentonite mixed with paraffin emulsion

Modified bentonite containing intercalated Ag⁺ and Cu²⁺ ions in a single grain was obtained in the same way as in Example 4, except that the obtained modified bentonite suspension was then diluted with demineralised water to a modified bentonite concentration of 20%, i.e. additional 1 dm³ of water was added. The total volume of the suspension was about 5 dm³. The dilution process was carried out in the same mixer, where the ion exchange occurred. Then 5 dm³ of paraffin emulsion with a paraffin content of 20% was added to the mixer. A pH regulator was added in the form of 100 ml of 20% aqueous acetic acid solution to reduce the viscosity. The addition of acetic acid had a positive effect on the mixing of the paraffin emulsion with the modified bentonite. The mixing process was conducted for 1 hour. Then the whole was left for 1 hour without mixing.

The thus obtained aqueous composition of modified bentonite, paraffin emulsion and pH regulator with a concentration of about 20% can be applied by coating with a rod, air knife or roller on packaging papers in order to obtain paper with biocidal properties. This coating method is particularly suitable for papers with a basis weight above 60 g/m2.

### EMBODIMENT 6 - bentonite paste

Dry powder of activated bentonite was sieved on the sieve 400, with a mesh size below 33 microns. Next the bentonite was weighed, and 600 g of activated bentonite was added at once (as one portion) to a stirring tank with a thermostatic jacket (heating/cooling jacket) following the introduction 5 liters of distilled water (conductivity less than 5 microsiemens). All the process for paste preparation was carried out at about 25°C provided by the heating/cooling jacket. Water together with the powder of activated bentonite was stirred, and a highly viscous paste of concentration about 12% was obtained in the tank. The tank content was stirred at 0,5 rps (revolution per second). The stirring was carried out till a substantially homogenous suspension was obtained, without visible clumps of unwet bentonite powder - to this end, the stirring can be preferably conducted for 30 to 60 minutes. Next, two water solutions of metal nitrate, i.e., silver (I) nitrate (V) and copper (II) nitrate (V) were prepared, each solution of volume 500 ml. The first solution comprising 0,036 mol of silver (I) nitrate (V) (providing addition, into the suspension, of 0,06 mol of silver (I) ions per 1 kg of bentonite powder), and the second solution comprising 0,24 mol of copper (II) nitrate (V) (providing addition, into the suspension, of 0,4 mol of copper (II) ions per 1 kg of bentonite powder). The solutions were simultaneously fed with peristaltic pumps at a flow rate of 0,4 ml/s, into the tank with the continuously stirred suspension. The feeding system was configured so that the metal cations, Ag⁺ and Cu²⁺ were introduced into the tank at a certain distance from each other, and the stirring process was affected efficiently to quickly mix the bentonite powder suspension with both the metal nitrates. After all the amount of metal nitrates was added into the tank, the suspension in the tank was continuously stirred for about 30 minutes. Preferably the used stirring system should provide good primary and secondary circulation. The obtained paste was a viscous suspension of 10% concentration of modified bentonite (bentonite dry mass). The obtained paste was a ready-to-use product, optionally with a further dilution of the paste, if desirable. The obtained paste may be applied onto various substrates, for example, paper.

### EMBODIMENT 7 - bentonite paste with addition of silver (I) chloride (AgCl)

The paste was prepared similarly to Embodiment 6, however, different solutions of metal nitrates were feed into the tank, after mixing 600g of activated bentonite with 5 liters of distilled water for about 30 - 60 minutes at 25°C. First solution comprised 0,036 mol of silver (I) nitrate (V) (i.e., addition of 0,06 mol AgNOs per 1 kg of bentonite) and 0,012 mol of silver (I) chloride (i.e. addition of 0,02 mol of Ag ions per 1 kg of bentonite). The first solution was admixed with 0,01 mol of nitric (V) acid (HNO₃) which may be added as 12,5 ml of 5% HNO₃ (aqueous solution)). AgCl forms a suspension in the first solution (as it is insoluble in this solution), and the mentioned addition of HNO₃ affects pH decrease, since in this amount HNO₃ cannot effect AgCl dissolution. The second solution comprised 0,024 mol of zinc (II) nitrate (V) (i.e. addition of 0,4 mol of zinc (II) ions per 1 kg of bentonite). The first solution and second solution were fed into the tank simultaneously, in the same manner as in Embodiment 7. After all the amounts of the above solutions were added into the tank, the suspension in the tank was continuously stirred for about 2 hours. Preferably the used stirring system should provide good primary and secondary circulation. The obtained paste was a viscous suspension of 10% concentration of modified bentonite (bentonite dry mass). The obtained paste was a ready-to-use product, optionally with a further solvent (e.g. water) dilution of the paste, if desirable. The paste may be applied onto various substrates such as paper, or other substrates. The addition of AgCl - described above, provides an increased level of intercalation bentonite grains with silver ions (Ag⁺), compared with the modification without AgCl addition. It is believed that AgCl interacts with nitrate groups to form silver nitrate, in an acidic environment. Obtained this way, the silver nitrate, further modifies (intercalates) the bentonite grains, thus increasing the Ag-intercalation level of the bentonite grains. The addition of AgCl provides the above results, even at lower bentonite concentration, i.e. below 2% of bentonite.

### EMBODIMENT 8 - paste rod -coating on cellulose substrate

600g of paste (prepared according to Embodiment 6) comprising 10% of the modified bentonite was diluted with distilled water to obtain the bentonite concentration of 8%. This concentration provides a paste consistency that is suitable for rod-coating. The dilution was performed in the tank with a stirrer, at a stirring speed of 0,5 rps, whilst feeding the paste with 1,5 liters of distilled water. The water was fed in portions of 250 ml each. Next, the obtained substantially homogenous slurry of low viscosity was transferred into the feeding system of the rod-coater device, from where the paste was fed, via a transfer roll, onto the coated paper substrate. The excess of the paste was collected with Meyer's rod. The coating formed on the substrate had a grammage of 6g/m² of dry mass. After being coated, the paste (a slurry) was dried with hot air delivered in counter-current with respect to the paper sheet movement to obtain a drying degree of not less than 90%. The dried paper sheet was rolled. The obtained paper was ready-to-use.

In the same way, the paste of Embodiment 7 can be applied on a cellulosic substrate.

### EMBODIMENT 9 - application of the modified bentonite with spraying nozzle

600g of paste (prepared according to Embodiment 6) comprising 10% of the modified bentonite was diluted with distilled water to obtain the bentonite concentration of 3%, this concentration provides a consistency that is suitable for spraying. The dilution was performed in the tank with a stirrer, at a stirring speed of 0,5 rps (revolution per second). During the stirring, 15 liters of distilled water was fed, into the tank, in a small water stream, the feeding time was about 5 minutes. After introducing all the amount of water, the slurry in the tank was further stirred, for about 10 minutes. Next, a substantially homogenous suspension of low viscosity was obtained. The suspension was transferred into the feeding system of the spray nozzle device from where, through the nozzles set, by using compressed air, the slurry was sprayed onto the substrate (cellulose tissue sheet of grammage of 1,6g/mm²), at the spraying width enabling the ribbon to be evenly sprayed. The obtained coating had a grammage of 1g/m² of dry mass. After being coated, the slurry was dried with hot air delivered in counter-current with respect to the sheet movement to obtain a drying degree of not less than 90%, (instead, the obtained coating may be dried on the Yankee cylinder). The dried sheet was rolled. The obtained tissue paper was ready-to-use.

In the same way, the paste of Embodiment 7 can be applied on a cellulosic substrate.

The obtained substrate was investigated according to standards: EN ISO 21149:2017-07 and EN ISO 16212:2017-08. To this end there were prepared three samples of diameters 60 mm each. The total time of assay: 10 days, total contact duration 4 hours +/-1 minutes; temp. of contact 20 +/-1 °C; neutralizer: bouillon D/E (Difco); incubation temp. 37 +/- 1 °C; bacteria strains used: Klebsiella pneumoniae ATCC 4352. The obtained result - bacterial reduction (IgN₀-IgN_{A}) was 6,96, which confirmed very good antibacterial properties of the obtained coating with the modified bentonite.

## Claims

1. A modified bentonite comprising intercalated bentonite grains having a size of at least 200 nm, wherein each of the intercalated bentonite grains is intercalated with at least two different metal ions selected from the group consisting of the following pairs of ions: Ag⁺ and Cu²⁺ ions, Ag⁺ and Zn²⁺ ions, Ag⁺ and Ni²⁺ ions.

2. The modified bentonite according to claim 1, wherein the modified bentonite comprises at least two ingredients selected from the group consisting of: at least 0.001 mole of silver (I) ions per 1 kg of the bentonite, at least 0.001 mole of zinc (II) ions per 1 kg of the bentonite, and at least 0.001 mole of copper (II) ions per 1 kg of the bentonite.

3. The modified bentonite according to any of the preceding claims, wherein the intercalated bentonite grains have a diameter from 200 nm to 50 µm.

4. A composition comprising the modified bentonite according to any of the preceding claims, further comprising at least one of: zeolite grains, cellulose fibres and water.

5. The composition according to claim 4, comprising the zeolite grains of which each zeolite grain contains in its structure at least two different metal ions selected from the group consisting of silver ions (Ag+) and dipositive metal ions (Me²⁺).

6. The composition according to claim 5, comprising the zeolite grains of which each zeolite grain contains in its structure at least two different metal ions selected from the group consisting of: Ag⁺, Cu²⁺, Zn²⁺, Sn²⁺, Co²⁺, Ni²⁺, Bi²⁺ and Zr²⁺.

7. The composition according to any of claims 4 to 6, comprising the modified bentonite grains and the zeolite grains in a mass ratio of the modified bentonite grains : zeolite grains from 50:1 to 5:1.

8. The composition according to any of claims 4 to 7, comprising cellulose fibres having a length from 50 to 500 nm.

9. The composition according to claim 8, comprising 5 to 20% of the cellulose fibres with respect to the mass of the modified bentonite.

10. The composition according to any of claims 8 or 9, comprising water and further comprising a cationic polyelectrolyte in an amount effective to obtain a neutral electrokinetic potential of the composition.

11. The composition according to claim 10, comprising at least one cationic polyelectrolyte selected from the group consisting of polyacrylamide and cationic starch.

12. The composition according to claim 10 or 11, comprising from 0.5 to 10 wt.% of the modified bentonite with respect to the total mass of the composition.

13. A method for manufacturing the modified bentonite according to any of claims from 1 to 8, the method comprising: mixing each of a plurality of bentonite grains having a size of at least 200 nm, with water solutions of two different water-soluble metal nitrates (V) selected from the group consisting of the following pairs of nitrates: silver (I) nitrate (V) and copper (II) nitrate (V), silver (I) nitrate (V) and zinc (II) nitrate (V), silver (I) nitrate (V) and nickel (II) nitrate (V), to obtain the intercalated bentonite grains, wherein the bentonite grains used as a substrate for the mixing are bentonite grains activated with sodium and/or potassium ions in a form of an aqueous suspension, wherein the bentonite concentration in the aqueous suspension is preferably from 5 to 30 wt.%.

14. The method according to claim 13, wherein the mixing step comprises mixing two different water-soluble metal nitrates (V) selected from the group consisting of: 0.005 mole of silver (I) nitrate (V) per 1 kg of the bentonite, 0.005 mole of zinc (II) nitrate (V) per 1 kg of the bentonite, and 0.005 mole of copper (II) nitrate (V) per 1 kg of the bentonite.

15. The method according to any of claims from 13 to 14, further comprising: washing and dewatering the intercalated bentonite grains to obtain dried intercalated bentonite grains, and next mixing the dried intercalated bentonite grains with an aqueous mixture of cellulose fibres of length from 50 to 500 nm and polyelectrolyte, wherein the aqueous mixture comprises 5 to 20% of the cellulose fibres and 0.1 to 1 % of the polyelectrolyte, and wherein the method further comprises: diluting the obtained modified bentonite with water, to a concentration of the modified bentonite in this mixture from 0.1 to 2%, and applying the diluted modified bentonite mixture to cellulosic surfaces, by spraying or by roller-coating.

## Patentansprüche

1. Modifizierter Bentonit, der interkalierte Bentonitkörner mit einer Größe von zumindest 200 nm umfasst, wobei jedes der interkalierten Bentonitkörner mit zumindest zwei verschiedenen Metallionen interkaliert ist, die ausgewählt sind aus der Gruppe bestehend aus den folgenden Ionenpaaren: Ag⁺- und Cu²⁺-Ionen, Ag⁺- und Zn²⁺-Ionen, Ag⁺- und Ni²⁺-Ionen.

2. Modifizierter Bentonit nach Anspruch 1, wobei der modifizierte Bentonit zumindest zwei Bestandteile umfasst, die ausgewählt sind aus der Gruppe bestehend aus: zumindest 0,001 Mol Silber(I)-Ionen pro 1 kg Bentonit, zumindest 0,001 Mol Zink(II)-Ionen pro 1 kg Bentonit und zumindest 0,001 Mol Kupfer(II)-Ionen pro 1 kg Bentonit.

3. Modifizierter Bentonit nach einem der vorhergehenden Ansprüche, wobei die interkalierten Bentonitkörner einen Durchmesser von 200 nm bis 50 µm aufweisen.

4. Zusammensetzung, umfassend den modifizierten Bentonit nach einem der vorhergehenden Ansprüche, ferner umfassend zumindest eines von: Zeolithkörnern, Zellulosefasern und Wasser.

5. Zusammensetzung nach Anspruch 4, umfassend die Zeolithkörner, von denen jedes Zeolithkorn in seiner Struktur zumindest zwei verschiedene Metallionen enthält, die ausgewählt sind aus der Gruppe bestehend aus Silberionen (Ag+) und dipositiven Metallionen (Me²⁺).

6. Zusammensetzung nach Anspruch 5, umfassend die Zeolithkörner, von denen jedes Zeolithkorn in seiner Struktur zumindest zwei verschiedene Metallionen enthält, die ausgewählt sind aus der Gruppe bestehend aus: Ag⁺, Cu²⁺, Zn²⁺, Sn²⁺, Co²⁺, Ni²⁺, Bi²⁺ und Zr²⁺.

7. Zusammensetzung nach einem der Ansprüche 4 bis 6, umfassend die modifizierten Bentonitkörner und die Zeolithkörner in einem Massenverhältnis der modifizierten Bentonitkörner : Zeolithkörner von 50:1 bis 5:1.

8. Zusammensetzung nach einem der Ansprüche 4 bis 7, umfassend Cellulosefasern mit einer Länge von 50 bis 500 nm.

9. Zusammensetzung nach Anspruch 8, umfassend 5 bis 20 % der Cellulosefasern in Bezug auf die Masse des modifizierten Bentonits.

10. Zusammensetzung nach einem der Ansprüche 8 oder 9, umfassend Wasser und ferner umfassend einen kationischen Polyelektrolyten in einer Menge, die wirksam ist, um ein neutrales elektrokinetisches Potential der Zusammensetzung zu erhalten.

11. Zusammensetzung nach Anspruch 10, umfassend zumindest einen kationischen Polyelektrolyten, der ausgewählt ist aus der Gruppe bestehend aus Polyacrylamid und kationischer Stärke.

12. Zusammensetzung nach Anspruch 10 oder 11, umfassend 0,5 bis 10 Gew.-% des modifizierten Bentonits in Bezug auf die Gesamtmasse der Zusammensetzung.

13. Verfahren zur Herstellung des modifizierten Bentonits nach einem der Ansprüche 1 bis 8, wobei das Verfahren Folgendes umfasst: Mischen von jedem aus einer Vielzahl von Bentonitkörnern mit einer Größe von zumindest 200 nm mit Wasserlösungen von zwei verschiedenen wasserlöslichen Metallnitraten (V) ausgewählt aus der Gruppe bestehend aus den folgenden Nitratpaaren: Silber(I)-Nitrat (V) und Kupfer(II)-Nitrat (V), Silber(I)-Nitrat (V) und Zink(II)-Nitrat (V), Silber(I)-Nitrat (V) und Nickel(II)-Nitrat (V), um die interkalierten Bentonitkörner zu erhalten, wobei die Bentonitkörner, die als Substrat für das Mischen verwendet werden, Bentonitkörner sind, die mit Natrium- und/oder Kaliumionen in einer Form einer wässrigen Suspension aktiviert sind, wobei die Bentonitkonzentration in der wässrigen Suspension bevorzugt 5 bis 30 Gew.-% ist.

14. Verfahren nach Anspruch 13, wobei der Mischschritt Mischen von zwei verschiedenen wasserlöslichen Metallnitraten (V) umfasst, die ausgewählt sind aus der Gruppe bestehend aus: 0,005 Mol Silber(I)-Nitrat (V) pro 1 kg Bentonit, 0,005 Mol Zink(II)-Nitrat (V) pro 1 kg Bentonit und 0,005 Mol Kupfer(II)-Nitrat (V) pro 1 kg Bentonit.

15. Verfahren nach einem der Ansprüche 13 bis 14, ferner umfassend: Waschen und Entwässern der interkalierten Bentonitkörner, um getrocknete interkalierte Bentonitkörner zu erhalten, und als nächstes Mischen der getrockneten interkalierten Bentonitkörner mit einer wässrigen Mischung aus Cellulosefasern mit Länge von 50 bis 500 nm und Polyelektrolyt, wobei die wässrige Mischung 5 bis 20 % der Cellulosefasern und 0,1 bis 1% des Polyelektrolyten umfasst, und wobei das Verfahren ferner Folgendes umfasst: Verdünnen des erhaltenen modifizierten Bentonits mit Wasser, auf eine Konzentration des modifizierten Bentonits in dieser Mischung von 0,1 bis 2 % und Aufbringen der verdünnten modifizierten Bentonitmischung auf Celluloseoberflächen durch Sprühen oder Walzenbeschichten.

## Revendications

1. Bentonite modifiée comprenant des grains de bentonite intercalés comportant une taille d'au moins 200 nm, chacun des grains de bentonite intercalés étant intercalé avec au moins deux ions métalliques différents choisis dans le groupe constitué par les paires d'ions suivantes : les ions Ag⁺ et Cu²⁺, les ions Ag⁺ et Zn²⁺ ions, les ions Ag⁺ et Ni²⁺.

2. Bentonite modifiée selon la revendication 1, ladite bentonite modifiée comprenant au moins deux ingrédients choisis dans le groupe constitué par : au moins 0,001 mole d'ions d'argent (I) pour 1 kg de la bentonite, au moins 0,001 moles d'ions de zinc (II) pour 1 kg de bentonite, et au moins 0,001 mole d'ions de cuivre (II) pour 1 kg de bentonite.

3. Bentonite modifiée selon l'une quelconques des revendications précédentes, lesdits grains de bentonite intercalés comportant un diamètre allant de 200 nm à 50 µm.

4. Composition comprenant la bentonite modifiée selon l'une quelconques des revendications précédentes, comprenant en outre au moins un élément parmi : les grains de zéolite, les fibres de cellulose et l'eau.

5. Composition selon la revendication 4, comprenant les grains de zéolite parmi lesquels chaque grain de zéolite contient dans sa structure au moins deux ions métalliques différents choisis dans le groupe constitué par les ions d'argent (Ag+) et des ions métalliques dipositifs (Me²⁺).

6. Composition selon la revendication 5, comprenant les grains de zéolite parmi lesquels chaque grain de zéolite contient dans sa structure au moins deux ions métalliques différents choisis dans le groupe constitué par : Ag⁺, Cu²⁺, Zn²⁺, Sn²⁺, Co²⁺, Ni²⁺, Bi²⁺ et Zr²⁺.

7. Composition selon l'une quelconque des revendications 4 à 6, comprenant les grains de bentonite modifiée et les grains de zéolite dans un rapport pondéral des grains de bentonite modifiée:grains de zéolite allant de 50:1 à 5:1.

8. Composition selon l'une quelconque des revendications 4 à 7, comprenant des fibres de cellulose comportant une longueur allant de 50 à 500 nm.

9. Composition selon la revendication 8, comprenant 5 à 20 % des fibres de cellulose par rapport à la masse de la bentonite modifiée.

10. Composition selon l'une quelconque des revendications 8 ou 9, comprenant de l'eau et comprenant en outre un polyélectrolyte cationique en une quantité efficace pour obtenir un potentiel électrocinétique neutre de la composition.

11. Composition selon la revendication 10, comprenant au moins un polyélectrolyte cationique choisi dans le groupe constitué par le polyacrylamide et l'amidon cationique.

12. Composition selon la revendication 10 ou 11, comprenant de 0,5 à 10 % en poids de la bentonite modifiée par rapport à la masse totale de la composition.

13. Procédé permettant la fabrication de la bentonite modifiée selon l'une quelconque des revendications 1 à 8, le procédé comprenant : le mélange de chacun d'une pluralité de grains de bentonite comportant une taille d'au moins 200 nm, avec des solutions aqueuses de deux nitrates (V) métalliques solubles dans l'eau différents choisis dans le groupe constitué par les paires de nitrates suivantes : le nitrate (V) d'argent (I) et le nitrate (V) de cuivre (II), le nitrate (V) d'argent (I) et le nitrate (V) de zinc (II), le nitrate (V) d'argent (I) et le nitrate (V) de nickel (II), pour obtenir les grains de bentonite intercalés, lesdits grains de bentonite intercalés utilisés comme substrat pour le mélange étant des grains de bentonite activés avec des ions sodium et/ou potassium sous la forme d'une suspension aqueuse, ladite concentration de bentonite dans la suspension aqueuse étant de préférence de 5 à 30 % en poids.

14. Procédé selon la revendication 13, ladite étape de mélange comprenant le mélange de deux nitrates (V) métalliques solubles dans l'eau différents choisis dans le groupe constitué par : 0,005 mole de nitrate (V) d'argent (I) pour 1 kg de la bentonite, 0,005 mole de nitrate (V) de zinc (II) pour 1 kg de la bentonite, 0,005 mole de nitrate (V) de cuivre (II) pour 1 kg de la bentonite.

15. Procédé selon l'une quelconques des revendications 13 à 14, comprenant en outre : le lavage et la déshydratation des grains de bentonite intercalés pour obtenir des grains de bentonite intercalés séchés, et ensuite le mélange des grains de bentonite intercalés séchés avec un mélange aqueux de fibres de cellulose d'une longueur allant de 50 à 500 nm et d'un polyélectrolyte, ledit mélange aqueux comprenant 5 à 20 % des fibres de cellulose et 0,1 à 1 % du polyélectrolyte, et ledit procédé comprenant en outre : la dilution de la bentonite modifiée obtenue avec de l'eau, jusqu'à une concentration de la bentonite modifiée dans ce mélange de 0,1 à 2 %, et l'application du mélange de bentonite modifiée diluée sur des surfaces cellulosiques, par pulvérisation ou enduction au rouleau.
